# EUROPEAN PATENT APPLICATION

(11) **EP 4 840 409 A2**
(43) Date of publication of application: **18.11.2026**
(21) Application number: 26188037.1
(22) Date of filing: 23.08.2019
(51) Int. Cl.: A61B 17/12

(54) **A FAULT-TOLERANT ENDOVASCULAR INFLATION DEVICE**

(30) Priority: 24.08.2018 DK PA201800507
(62) Document number(s) of the earlier application(s) in accordance with Art. 76 EPC: 19759341.1 / 3 840 817
(71) Applicant: Neurescue ApS, 1106 København K (DK)
(72) Inventor: FROST, Habib, 1106 København K (DK); HULDT, Olof, 1106 København K (DK); NØRLØV, Mads, Bundgaard, 1106 København K (DK)
(74) Representative: Inspicos P/S

(57) **Abstract**

An endovascular device for providing at least partial occlusion in a blood vessel in a subject, e.g. for improving Cardiopulmonary Resuscitation. The device comprises a balloon catheter to be inserted in a blood vessel for inflation therein. To increase the flexibility and safety in use of the device and to enable use under controlled and less controlled environments, the device comprises a first interfacing means configured to connect to a first manually operated inflation means and being in fluid communication with a junction; a second interfacing means configured to connect to a second power controlled inflation means and also being in fluid communication with the junction, and an electronic control unit configured to receive the occlusion parameter from the sensor and to provide an instruction set for manually operated inflation or for automatically operated inflation based on the occlusion parameter.

## Description

The disclosure relates an endovascular device for providing at least partial occlusion in a blood vessel in a subject. By the at least partial occlusion, the flow of blood in the blood vessel is limited or prevented. In the following, at "least partial occlusion" is for simplicity referred to simply as "occlusion". The device comprises an elongated body extending between a proximal end and a distal end. The distal end is configured by its shape and size such that it can be inserted into the blood vessel in which occlusion is desired. An inflatable member is formed about the elongated member and configured to expand upon receipt of a fluid medium by operation of an inflation means, and a sensor is arranged for sensing an occlusion parameter in the blood vessel. The disclosure further relates to a method for providing occlusion in a blood vessel. Applicant's technically related previous US 10,143,789 and WO 2017/093483 are hereby incorporated by reference herein in their entirety.

### BACKGROUND

Cardiovascular disease contributes 30.9% of global mortality. Currently only 1 out of 10 survive a cardiac arrest to hospital discharge. It is responsible for higher mortality rates than any other disease in industrialized countries, and three-quarters of non-infectious mortality in developing countries. In the US there are around 350.000 cardiac arrests outside of hospitals; and approximately as many inside hospitals. The potential for improvement is massive.

By the early 1970s, CPR (Cardiopulmonary Resuscitation), defibrillation, and prehospital care were all in place. The introduction of automated defibrillation units (AED) expanded the possibility for prehospital treatment of cardiac arrest, and the first AED was successfully put to use by paramedics in Brighton in 1980. In spite of this, our current best practice only has the ability to achieve resuscitation, return of spontaneous circulation (ROSC), for around 25-30% of patients both in pre-hospital and in-hospital settings.

Devices and methods exist for providing occlusion in blood vessels. Such devices are used in resuscitation or in cardiac arrest to increase return of spontaneous circulation (ROSC) and expand the time window of intervention.

The existing devices require fluoroscopy guidance and the procedure of operation is complicated by the large immobile fluoroscopy machines. Accordingly, occlusion procedures are typically only carried out in controlled environments, particularly in hospitals, and only by highly trained specialist medical staff.

Medical catheters are used in a wide range of procedures, often including an inflatable member configured to provide a specific therapeutic effect or redistributing a medium. Catheterization of these inflatable members must accommodate a wide range of sizes. The inflatable member is predominantly a compliant balloon that can stretch to a wide range of final filling diameters to facilitate a thin-profiled catheter that can be used e.g. to occlude a blood vessel and which can match blood vessels in a broad range of sizes.

The size may, as an example range from 10-32 mm for e.g. aorta or vena cava occlusion. For increased safety, there is a desire for automation to prevent user error, i.e. when the device is used in an unintended way and based only on human senses and manual user interaction, there is a risk to the patient. Further, when imaging modalities, e.g. based on fluoroscopy machines, are unavailable, an increased risk may result.

Even though automatic systems in some ways may improve the safety, automatic systems may also have weaknesses. Electronics or software may fail and therefore constitute a risk to the patient or prevent completion of the procedure. For that reason, a technical prejudice is sometimes experienced against automation, and the trained medical staff tends not to rely on automation, particularly in connection with high-risk procedures.

In an emergency situation, where time is critical, use of advanced imaging capabilities is often not an option since it requires time-consuming procedures. Use of fluoroscopy, CT, or MR scanning for intravascular catheterization may therefore not be an option in an emergency situation.

It is desirable to provide a solution to the problem of safe medical intervention also when the exact diameter of the blood vessel is unknown, or when imaging is not an option. It is also desirable to allow medical intervention in an emergency situation or when a complete repertoire of devices is unavailable.

Emergency situations include among other traumatic bleedings, non-traumatic bleedings, traumatic cardiac arrest and non-traumatic cardiac arrest. However, the use of a compliant balloon blindly necessitates supraphysiological inflation pressures, defined as an arterial pressure which is higher than 129 mmHg in the 2017 AHA guidelines. This pressure is necessary for expanding the balloon material beyond the flaccid-state circumference; the balloon pressure has to counteract the physiological pressures inside the compartment and, in addition to this, stretch the compliant material beyond the flaccid-state circumference to the desired final circumference. These pressures can exceed the pressure which is desired and which is safe for the intended procedure in a blood vessel, especially in a vessel already affected by a pathological mechanism, e.g. a traumatic tear, accumulated calcification, clot, embolism or blood vessel constriction.

Excess pressure can lead to blood vessel rupture or balloon rupture when using a standard catheter. Such risks have been described in 'Resuscitative endovascular balloon occlusion of the aorta: rupture risk and implications for blind inflation by Wasicek PJ, et al. Trauma Surg Acute Care Open 2018' as well as in numerous case reports. Furthermore, the state of the art is limited in lack of feedback in being able to distinguish and verify a correct positioning and successful therapeutic intervention without advanced imaging guidance, e.g. the insertion, inflation, deflation and extraction of a catheter. These imaging techniques also currently require a multi-year specialization in addition to a medical degree to be able to correctly interpret the images which limits the availability of these procedures and leaves a residual risk to the patient in the margin for error during the interpretation. More portable techniques including ultrasound cannot be used reliable above or near bony structures, such as the chest cage and need to be interpreted as well. Even when such imaging is available, the image interpretation and manual filling and expansion of e.g. a balloon material beyond the flaccid-state circumference with a high pressure leaves a risk of errors, particularly in the hand of the user with low sensitivity and high inter-user variability. This leads to risks of tissue damage if the balloon is overinflated and has an excessive pressure and/or balloon stretch, and potential loss of therapeutic value if the harm is not correctly identified. In the known art, the pressure inside a balloon can be limited by a simple mechanical pressure relief valve or electronic pressure-limiter. This potential use as a failsafe against a supraphysiological pressure risk is unreliable with a standard compliant balloon due to the need for the valve or the limiter to be set to a supraphysiological pressure. This leads to a situation where inflation catheters are often inflated without feedback, fault-tolerance, or pressure control of where and when the inflation exercises its pressure. This increases the risk of damaging the patient's anatomy, especially in the stressful situation of an emergency condition. If only verified by human estimation, the balloon catheter could inadvertently, how rare these incidences might occur, end in e.g. an arterial branch of the aorta, in a venous vessel of the vascular system, in a dissection between layers of the aortic wall or in a tissue compartment outside of the vascular system; or raise the pressure so high at any of these locations that there is tissue damage or balloon rupture. Both tissue damage and balloon rupture may lead to serious injury to the patient.

### SUMMARY

It is an object of embodiments of the disclosure to provide an endovascular device which is attachable to the patient such that its interaction becomes independent of risk associated with user error or automation failure.

It is an object of embodiments of the disclosure to provide a device and a method by which ease-of-use, imaging-free use, and a built-in safety can mitigate for the risks involved in catheterization, including occlusion, and thereby enable methods for providing fault-tolerant and/or error-tolerant catheterization.

It is a further object to provide a device and a method by which the catheterization can be performed with low risk in near-community and hospital settings, in hands of users with minimal training, to thereby allow not only in-hospital specialists but also non-physicians and prehospital health care professionals and others to carry out such procedures.

In respect of the unmet needs in this field, the present disclosure, in a first aspect, provides an endovascular device for providing at least partial occlusion in a blood vessel in a subject. The device comprises an elongated body extending between a proximal end and a distal end. The distal end is shaped and sized to enable its insertion into the blood vessel. The device comprises an inflatable member formed about the elongated member and configured to expand upon receipt of a fluid medium by operation of an inflation means. The device further comprises a sensor for sensing an occlusion parameter in the blood vessel, the occlusion parameter being a parameter which can characterize a degree of occlusion in the blood vessel.

The device comprises:
- an inflation conduit providing fluid communication between the inflatable member and a junction,
- a first interfacing means configured to connect to a first manually operated inflation means and being in fluid communication with the junction;
- a second interfacing means configured to connect to a second power controlled inflation means and being in fluid communication with the junction, and
- an electronic control unit configured to receive the occlusion parameter from the sensor and to provide an instruction set for manually operated inflation or for automatically operated inflation based on the occlusion parameter.

By the junction and the first and second interfaces, use of two separate and different inflation means becomes possible. In that way:
The device becomes suitable for manual procedures by connection of a manually operated inflation means to the first interface. This can be selected either when the automatic features fail, when the operator prefers manual operation, and for manual verification of balloon emptying or empty balloon status at the end of a procedure before catheter extraction.

The device becomes suitable for automatic procedures by connection of a power controlled inflation means to the second interface. This can be selected when the user lacks sufficient skills to operate manually or when increased safety is desired.

Further, since the manual operation and the automatic operation takes place through different interfaces, the presence of one inflation means does not prevent the presence of another inflation means. That increases the fail safe of the system and allows the user to shift between automatic mode and manual mode during operation. It further allows the user to shift e.g. from manual to automatic mode should the manual inflation means become malfunctioning, vice versa. Accordingly, it provides a more fault tolerant device.

Accordingly, the disclosure provides an endovascular device which is attachable to the patient such that its interaction becomes independent of risk associated with user error or automation failure since in both cases, the user can select an alternative mode of operation. Additionally, the combination between information for manual operation and control for automatic inflation enables ease-of-use and optionally imaging-free use.

The elongated body may particularly be a catheter structure defining at least one conduit throughout its length. The elongated body extends from the proximal end to the distal end. Herein, the proximal end is away from the individual into which the elongated body is inserted, and the distal end is that end which is inserted into the blood vessel,

The inflatable member may particularly be a balloon made of an elastically deformable polymer material and configured to expand when filled with a fluid medium from an inflation means. The fluid medium could be gaseous or viscous. A most reliable pressure and thus occlusion may be obtained by a liquid fluid medium, e.g. saline.

The sensor which is configured to sense an occlusion parameter in the blood vessel could be a pressure sensor, or a flow sensor or similar kind of sensor. In one embodiment, the sensor is configured to sense a pressure which can indicate occlusion state, and in another embodiment, the sensor is an ultra sound sensor configured to indicate a flow and thus a status of the occlusion.

The inflation conduit enables a fluid flow between the inflatable member and a junction, and the first interfacing means can connect to a first manually operated inflation means and it is in flow communication with that junction. The second interfacing means can connect to a second power controlled inflation means and it is also in fluid communication with the junction. This allows two alternative ways of inflation of the inflatable member.

The electronic control unit receives the occlusion parameter from the sensor and provides therefrom a set of instructions either for a user to manually operate the manually operable inflation means or a set of instructions for the automatically operated inflation based on the occlusion parameter.

The set of instructions for manual operation may particularly be a signal which can be comprehended by human beings, e.g. a visual, audio, or tactile signal related to the operation of the manually operated inflation means, e.g. to increase or reduce a pumping speed or pressure etc. The set of instructions for the automatically operated inflation means may be a set of computer instructions, e.g. computer code, expressing control of the inflation, e.g. controlling a pump to increase or decrease pumping rate or pressure.

The device may be made in at least two distinct parts which can be assembled and optionally also be disassembled. The device may contain a fluid compartment. Particularly, the device may comprise a connector body and a controller body, wherein:
- the connector body is attachable to the controller body,
- the connector body is in fixed connection with the elongated body, and
- the power controlled inflation means is formed in the controller body.

This split provides several distinct advantages:
Firstly, it allows a sterilization method of the connector body independent on the sterilization method of the controller body.

Particularly since the controller body contains electronic components, it may require a different way of sterilization compared with the optimal way of sterilizing the connector body. In one embodiment of the device the fluid compartment is sterilized. In another embodiment the fluid compartment is pre-filled with fluid medium. In certain embodiments the internal fluid conduit(s) in the device, controller and/or connector is configured to be sterilized. These conduits may be made with silicone, ABS, PEBAX, PVC, Tygon and/or CFLEX. It may be an advantage to use radiation sterilization to ensure adequate penetrance for sterilization of an empty or pre-filled fluid compartment in the device or controller body (such a penetrance cannot be achieved by gaseous sterilization methods including Ethylene Oxide (ETO), Chlorine Dioxide, Hydrogen Peroxide or Hydrogen Peroxide plasma), whereas a gaseous sterilization can be used for sterilization of the connector body, since a gaseous sterilization protects possible MEMS-based sensor components in the device or connector from unacceptable radiation.

The vacuum during ETO is not acceptable with sterilization of batteries. Electronics could be negatively impaired by radiation sterilization, e.g. the magnetic field generated by electron beam sterilization.

A sensor necessitates different sterilization requirements of one or more distinct parts of the device, wherein one or more parts may be sterilized in different methods to facilitate sterilization of batteries or electronics.

In an embodiment of the device, the electronics inside the device may be configured to be sterilized with a gaseous/plasma or non-gaseous/plasma sterilization method by configuring the device with a partial encapsulation of the electronics and/or batteries, including encapsulation in a resin, including epoxy resin or other resins.

Gaseous/plasma sterilization methods may include autoclave steam, Ethylene Oxide (ETO), Chlorine dioxide, vaporized hydrogen peroxide or hydrogen peroxide plasma.

Radiation sterilization methods may include gamma ray and electron beam.

Problematic/unacceptable parameters: Autoclave steam reduces lifetime of embedded batteries. The vacuum during ETO is not acceptable for batteries. Chlorine dioxide has no adverse effect on electronics or batteries. Vacuum during Vaporized/Plasma hydrogen peroxide acceptable for embedded batteries. Furthermore, plasma may not be compatible with semiconductors. Gamma ray/Electron beam: The radiation can damage semiconductors.

In one embodiment of the device batteries and/or electronics embedded in the device, connector and/or controller may be encapsulated in resin, including epoxy resin, to facilitate one or more sterilization methods.

In an embodiment the device may be configured to have increased irradiation resistance by being configured the device with components that are radiation exposure hardened, including certified or tested parts by tests, e.g. by referring to the JEDEC publication JEP133C, *Guide for the Production and Acquisition of Radiation-Hardness-Assured Multichip Modules and Hybrid Microcircuits*.

The device may be configured to be sterilized with a gaseous/plasma substance, including ETO and Chlorine Dioxide (CD) Gas Sterilization, by being configured with adequate openings for adequate passage of the gaseous/plasma members.

In another embodiment the batteries and/or parts of the electronics may be made in a distinct part that is not sterilized, alternatively is sterilized by another method than other part(s) of the device.

In one embodiment of the device, the fluid compartment is made in a distinct part that is sterilized by another method than other part(s) of the device.

Secondly a split allows repetitive use of the controller body and single use of the connector body.

Thirdly, provided that the connector body and the controller bodies can also be disassembled, it allows separate disposal.

At least one of the first interfacing means and the second interfacing means could be formed in the connector body. By definition herein, the mentioning of at least one of two different entities is considered to mean one of the entities, the other one of the entities or both of the entities. I.e. in this case, at least one of the first interfacing means and the second interfacing means may also mean both of the first and the second interfacing means.

Since the connector body is in fixed connection with the elongated body and since the first interfacing means is formed in the connector body, the user may choose to use the connector body without using the controller body. In this way, the device can be operated completely without access to power and electronics by human operation of the manually operated inflation means connected to the first interfacing means. In extreme situations where electronic may not work, this is a safe way of ensuring durability.

The power controlled inflation means could be constituted by a piston pump, a peristaltic pump or any other kind of power driven pump. Additionally, the controller body may contain power supply means in the form of a battery, solar power cells, or any other means for establishing power. In one embodiment, the power-driven pump is driven by compressed gas, and the controller body comprises an ampule of compressed gas. This may increase the durability of the device against the impact of magnetic radiation.

Alternatively, at least one of the first interfacing means and the second interfacing means, e.g. both of them, could be formed in the controller body.

The device may comprise a storage body for storage of the fluid medium, the storage body may particularly be contained in the controller body. The storage body may be constituted by a sachet or ampule or syringe containing the fluid medium. The storage body may e.g. be releasably connected to the controller body to allow replacement or to allow instant fitting of a storage body in the controller body immediately prior to use. The device also allows for external storage bodies, intended for manual operation in the event of automation/electronic failure. Through the manual fluid ports in this embodiment, users may bypass the internal fluid storage of the controller and manually introduce the fluid independently of presence of the controller body.

The device may form an electronic sensor converter configured to receive a fluid signal from the elongated body, to convert the fluid signal to an electrical signal, and to communicate the electrical signal to the electronic control unit. The fluid signal may particularly be a signal indicating a pressure of fluid or a flow of fluid, and the electronic signal may particularly be a digital or analogue and electrically expressed indication of that pressure or flow. The sensor converter communicates the electrical signal to the electronic control unit, and it may particularly be located in the connector body.

The fluid signal could be received by the electronic sensor converter via a sensor conduit extending from an upstream location and the electronic sensor converter.

The upstream location may particularly be between the distal end and the inflatable member. At the upstream location, the sensor conduit may e.g. form an opening allowing pressure in the blood vessel to propagate into the sensor conduit and down to the electronic sensor converter.

To enable propagation of the pressure or to increase the precession of the pressure propagation, a purge structure may be provided to allow filling of the sensor conduit with a propagation medium, e.g. a liquid medium such as saline.

The purge structure may comprise an external access port configured to connect a purge fluid container. The external port may e.g. form a puncture or a connector for connecting a syringe. The external access port may particularly be provided in the connector body to thereby allow purging of the sensor independently of presence or functionality of the controller body.

The purge structure may alternatively, or additionally, comprise a confluence configured to establish fluid communication between the sensor conduit and the storage body.

The confluence may be configured for control by a pressure difference between pressure in the inflation conduit and pressure in the sensor conduit, such that it allows a fluid flow between the inflation conduit and the sensor conduit upon a pressure difference above a first threshold value and such that it prevents fluid flow between the inflation conduit and the sensor conduit upon a pressure difference below the first threshold value. A removable sheath may be configured to the size of the inflatable member and configured to prevent expansion of the inflatable member during operation of the inflation means to thereby trigger a flow via the confluence to the sensor conduit based on the pressure difference.

The confluence may be configured for control with an electronic valve, including an electronic solenoid valve, pinch valve and tube pinch valve.

The electronic sensor converter could be contained in the connector body. The connector body may form a first electric communication interface configured to electrically communicate with a second electric communication interface provided in the controller body. The communication interfaces may be constituted e.g. by phone jack plugs, e.g. by mini jack plugs, or wireless communication.

The connector body may form a first fluid communication interface configured to communicate fluid with a second fluid communication interface provided in the controller body. The first and second fluid communication interfaces thereby constitutes the second interfacing means which interfaces the inflatable member with a power controlled inflation means in the form of a pump housed in the controller body.

The first electric communication interface and the first fluid communication interface could be arranged to form a first mutual connection interface in the connector body. Likewise, the second electric communication interface and the second fluid communication interface could be arranged to form a second mutual connection interface in the controller body. In this embodiment the first and second mutual connection interfaces could be configured for establishing both electrical communication and fluid communication by joining the connector body and the controller body. Particularly the first and second mutual connection interfaces may establish the fluid and electrical communication simultaneously.

In one embodiment, a valve, puncture or a seal which can be ruptured is arranged to close at least one of the first and second fluid communication interfaces and the first and second mutual connection interfaces are configured to pierce, puncture or rupture that seal upon establishing electrical communication between the first and second electric communication interfaces such that fluid spillage is prevented until the connector body and controller body are joined.

The controller body may, likewise, contain the electronic control unit and be configured to receive the electrical signal via the second electric communication interface. In the electronic control unit, the electronic signal is converted into the instruction set for manual or automatic operation of the inflation means.

The connector body may contain power storage for powering the electronic sensor converter independently on the controller body to thereby make operation of the connector body independent on the controller body. Alternatively, the connector body may contain a power interface for receiving power from the controller body. The power interface may e.g. be included in the aforementioned first and second electric communication interfaces.

The electronic control unit may be configured to provide the instruction set such that it contains at least one of:
- a human signal for assisting human operator during operation of the manually operated inflation means, and
- an electronic control signal for controlling the power controlled inflation means.

The human signal may be configured to be received by a human operator. In a simple implementation, the information identifies when the degree of inflation is correct. In more advanced implementations, the information may identify other issues, e.g. related to correct or wrong location of the elongated body in the blood vessel. The human signal may be provided as a visually recognizable signal, an audio signal, or a tactile signal.

A visually recognizable signal could be provided by a lamp or screen display, an audio signal by a speaker submitting a tone or an audible instruction read by a computer voice, and the tactile signal could e.g. be shaking of the device.

The elongate body may have at least one distal port configured to sense at least one of the following: temperature; sound; infrared, distance, LIDAR, image sensors including ultrasound and X-ray, light, fluorescence, photoelectric effect; pressure; magnetism; flow, angular displacement, force, motion, inertia, electric impulses including ECG, EEG and EMG, glucose concentration, pO2, pCO2, SO2 or pH. The elongate body may incorporate active or passive RFID.

The proximal end may comprise a catheter connector or hub with a first interface configured for attaching the device to an automated inflation system, said first interface being further configured to be attachable to a fall back manual inflation means. The connector or hub may also be configured for access to the at least one distal port for relay of the sensor data or for relay of the sensor medium e.g. ports for access to fluid or an electronic signal. The disclosed embodiment may further comprise an electrical safety mechanism.

The inflatable member 5 may be stored inside a removable sheath narrowly enclosing the inflatable member while the inflatable is in a deflate state. In an embodiment, the electronic control unit is configured to be able to detect whether the inflatable member is inside the removable sheath by the electronic control unit first instructing a propagation of inflation medium to the inflation member and then sensing a corresponding occlusion parameter characteristic of the inflation member being enclosed inside the removable sheath. This detection may be used to ensure that zeroing of the occlusion parameter sensors, including pressure sensors, wherein the sensing would be a premature increase in pressure value compared to the amount of medium propagated to the inflation member. This would be useful to ensure zeroing of the occlusion parameter sensor is performed while the inflatable member is in atmospheric air, outside a blood vessel, while not yet inserted into a body. The removable sheath may comprise e.g. a peel-away sheath introducer.

The device may comprise a hose providing fluid communication between the inflation means and the storage body containing the inflation medium. The storage body, and/or the hose, and/or the inflation conduit may be made from polypropylene, polyethylene, Polyethylene terephthalate, Polyvinyl chloride, Polyethylene laminated polyethylene terephthalate, or is made from a synthetic polymer covered with a layer of metal.

In a second aspect, the disclosure provides a method for preparing a device according to the first aspect for use, the method comprises the step of connecting the connector body to the controller body.

In a third aspect, the disclosure provides a method for effecting at least partial reduction of blood supply to a part of the body of a mammal, the method comprising introduction of the endovascular device according to the first aspect via its distal end into a blood vessel of the mammal and advancing the inflatable member to a position in the mammal's vascular system via which blood is supplied to the part of the body and inflating the inflatable member to a degree which reduces blood flow from the position in the vascular system to the part of the body. Mammal herein may include human beings.

The method may comprise the step of selecting between use of a manual inflation process and an automatic inflation process, and based on the selection, using a manually operated inflation means based on the human signal or operating an automatically operated inflation means based on an electronic control signal. By means of an example, the manual operation may include the use of a syringe or similar manually operated pump and following the instructions provided by the control unit.

Blood flow from the position in the vascular system may be reduced to zero, or at least reduced sufficiently to comply with a desired redistribution of blood.

The position in the vascular system may particularly be in the descending aorta allowing, e.g. for a human being, redistribution to body areas above the inflatable member.

At least partial reduction in blood flow results in redistribution of cardiac output, and that can be continued until an increased blood supply is experienced in the brain and/or the heart, and the redistribution of the blood flow may be at least one measure undertaken in order to provide resuscitation or suspended state in the patient.

Chest compressions may be applied to the patient simultaneously with the resuscitation.

In one embodiment, the position in the vascular system is in an artery supplying one or more extremities or organs. And in one embodiment, the method is carried out to reduce or stop blood loss caused by arterial bleeding or arterial rupture.

The distal end of the endovascular device may be introduced via the femoral artery, preferably via a needle or cannula.

Further description and examples of methods which, within this disclosure may be carried out by use of the device according to the first aspect can be found in Applicant's technically related previous US 10,143,789 and WO 2017/093483 are hereby incorporated by reference herein in their entirety.

### LIST OF FIGURES

Figure 1 illustrates a perspective of an embodiment of said device;
Figure 2 illustrates an embodiment of said device, illustrating the connector body;
Figure 3 illustrates an embodiment of said device, illustrating a cross-sectional view of the distal end of the elongated body;
Figure 4 illustrates the embodiment of a connector body, seen from another angle, and the controller body;
Figure 5 illustrates the connector body, but without its cover in order to show the internal components in the connector body, along with the controller body without a cover. In this figure, the two bodies are connected as intended when operating the device;
Figure 6 illustrates the distal end of the elongated body; and
Figure 7 illustrates an embodiment of a connector body.

### DETAILED DESCRIPTION OF AN EMBODIMENT

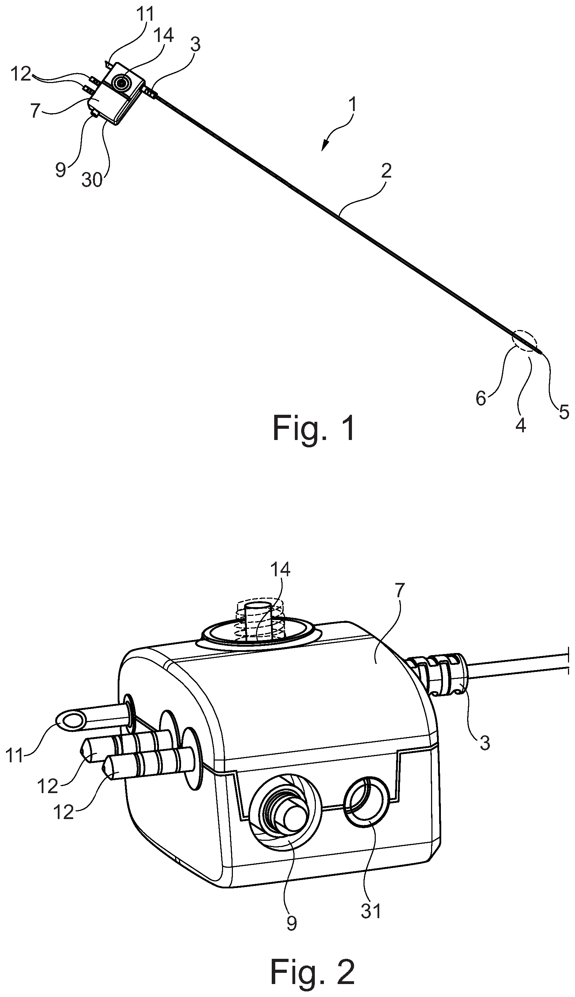

Fig. 1 illustrates the endovascular device **1**. The device comprises an elongated body **2** extending between a proximal end **3** and a distal end **4**, the distal end has a size and shape allowing its safe insertion into a blood vessel, e.g. into the aorta of a human being. For that purpose, the elongated body is terminated in a tip **5** which is made for safe insertion.

The device is configured to provide at least partly occlusion in the blood vessel by inflation of an inflatable member 6 within the blood vessel. The inflatable member **6** is formed about the elongated member and it is configured to be filled with a fluid medium and thereby expand. The fluid medium is received from an inflation means which is described in further details later. The fluid medium could be saline or a similar physiologically acceptable liquid.

The device comprises a sensor for sensing an occlusion parameter in the blood vessel*.* The sensor will be described in further details later, and may particularly be configured for sensing a blood pressure between the distal end and the inflatable member.

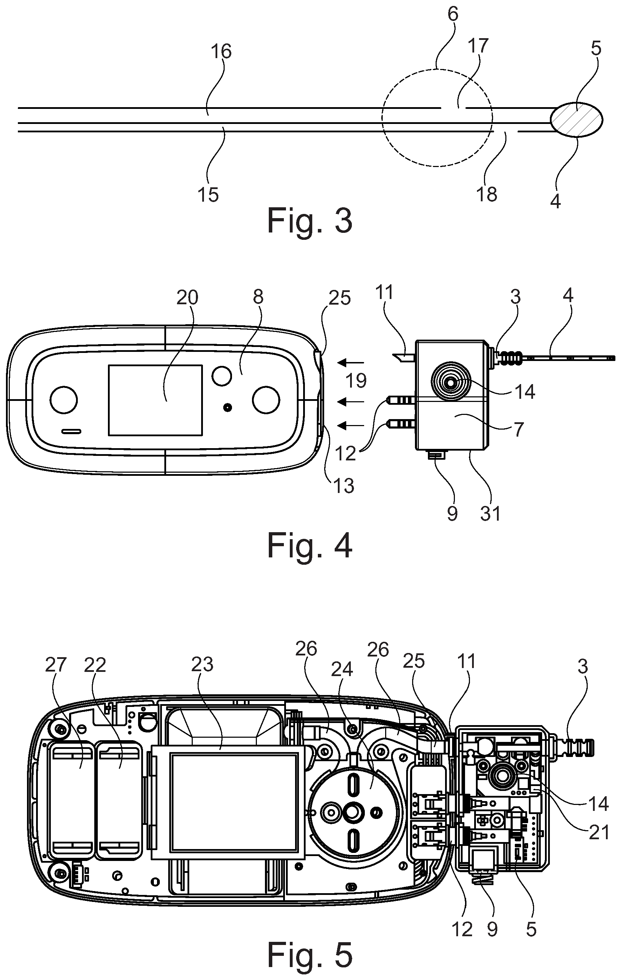

The device comprises a connector body **7** and a controller body **8**. The connector body **7** can be seen in Figs. 1 and 2, and in Figs. 4 and 5, the latter illustrating mutual functioning of the connector body **7** and the controller body **8**.

Figs. 1, 2, and 4 illustrates the connector body **7** seen from one side.

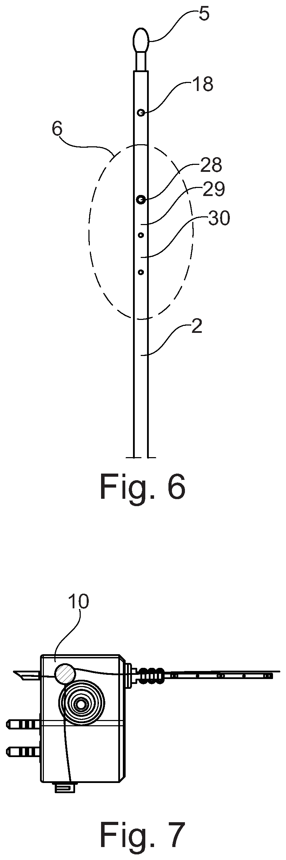

The connector body **7** comprises a first interfacing means **9**, configured to connect to a first manually operated inflation means (not shown). The first interfacing means **9** forms an external connection port and a fluid passage which connects the external connection port and a junction **10**. The junction can be seen in Fig. 7.

The connector body **7** further comprises a first fluid communication interface **11** constituting a part of the claimed second interfacing means. The first fluid communication interface forms an external connection port configured to connect to a second power controlled inflation means and a fluid connection between that external connection port and the junction **10**. The second power controlled inflation means may e.g. be a peristaltic pump, and it is located in the controller body **8**.

The connector body further forms a first electric communication interface **12** configured to electrically communicate with a second electric communication interface **13** provided in the controller body **8**.

The connector body **7** further comprises a purge structure comprising an external access port **14** configured to connect to a propagation medium container (not shown). The purge structure forms fluid connection between the connected propagation medium container and the sensor conduit **15** which is not shown in Figs. 1, 2 and 4 but which is illustrated in Fig. 3. The propagation medium container may particularly be a syringe, and the external access port **14** could be a puncture for sealing engagement with the syringe. The access port includes a valve function allowing fluid to flow from the external access port **14** to the sensor conduit **15**, but which prevents flow in the opposite direction from the sensor conduit **15** out through the external access port **14.**

The connector body **7** may be of a size making it portable, and preferably of a size making it handheld. Due to the fact that the endovascular device is supposed to be used in various environments, it is an aspect to make the connector body **7** robust to wear and tear. In an embodiment, the connector body **7** is a casing of hard plastic or any suitable material. In another embodiment, the connector body **7** is waterproof. When the connector body **7** is waterproof, the fluid connectors, i.e. the first fluid communication interface **11**, the access port **14**, and the first interfacing means **9** may be sealed, e.g. with a removable cap which prevents contamination and entrance of water or humidity into the connector body and into the conduits of the elongated body.

Fig. 3 illustrates the elongated body **2**, the inflatable member **6**, the sensor conduit **15**, and the upstream location **18.** At the upstream location, the sensor conduit forms an opening allowing pressure in the blood vessel to propagate into the sensor conduit and down to an electronic sensor converter to be discussed later.

In the illustrated embodiment, one single opening 18 is illustrated. In alternative embodiments, a plurality of openings may be provided at the upstream locations.

The inflation conduit **16** provides fluid communication between the junction **10** and thereby between the inflation means and the inflatable member. The upstream location **18** may particularly be between the distal end **4** and the inflatable member **6**.

Fig. 3 further illustrates an opening **17** between the inflation conduit **16** and the inner cavity within the inflatable member **6**.

Fig. 3 further illustrates the soft and rounded tip **5** which provides safe insertion into the blood vessel.

Fig. 4 illustrates the connector body **7** and the controller body **8**.

The connector body is attachable to the controller body by movement as indicated by the arrows **19**. The connector body is in fixed connection with the elongated body but can be released from the controller body. In that way, the connector body may be used as a stand alone product without the controller body, e.g. where automatic functions are not desired, and it can be used with the controller body where automatic functions are desired.

The controller body contains different electronic features and provides a user interface **20**.

Fig. 5 illustrates the connector body along with the controller, but exposed without an external cover.

In this view, the internal components of the connector body and the controller body are visible.

The connector body **7** comprises an electronic sensor converter **21** configured to receive a fluid signal through the sensor conduit **15**. The fluid signal represents pressure in the blood vessel above the inflatable member **6** and thereby represents an occlusion parameter.

The electronic sensor converter 21 converts the fluid signal to an electrical signal and communicates the electrical signal via the first electrical communication interface **12**.

The electrical signal transmitted via the first electrical communication interface **12** is received by the controller body **8** via the corresponding second electrical communication interface **13**.

In the controller body **8**, the electrical signal is transmitted to the electronic control unit **22** which, based on the electrical signal provides an instruction set for manual or automatic inflation.

The controller body **8** further comprises a storage body **23** containing a sufficient amount of a fluid medium for expansion of the inflatable member **6**. The fluid medium may particularly be saline or simply sterile water.

The controller body **8** further comprises a pump **24**, e.g. in the form of a peristaltic pump. The pump is connected between the storage body **23** and a fluid communication exit **25** via the pump tubing **26**, e.g. made of silicone. The fluid communication exit **25** is arranged and configured for communication with the first fluid communication interface **11** provided on the connector body **7**, the fluid communication exit 25 therefore forms a second fluid communication interface for communication with the first fluid communication interface and thereby forms part of the claimed second interfacing means, i.e. the first and second fluid communication interfaces defines the second interfacing means.

Additionally, the controller body **8** comprises a battery **27** allowing operation independent of external power.

To make the device suitable for storage over time to prevent diffusion, including diffusion of oxygen and water to and from the fluid system, one or more of the following features may be provided, since pump tubing materials are prone to diffusion, e.g. silicone tubing:
1) The material of the water bag may be configured to be diffusion resistant, e.g. by using PET/PE foil products, polypropylene, polyethylene, Polyethylene terephthalate, Polyvinyl chloride or Polyethylene laminated polyethylene terephthalate, or is made from a synthetic polymer covered with a coating or laminate of metal, hereunder including by aluminum coating.
2) The material of the pump tubing may be configured to be diffusion resistant, e.g. by using PET/PE foil products, polypropylene, polyethylene, Polyethylene terephthalate, Polyvinyl chloride or Polyethylene laminated polyethylene terephthalate, or is made from a synthetic polymer covered with a coating or laminate of metal, hereunder including by aluminum coating.
3) The fluid medium may be diffusion resistant. It may e.g. comprise or contain Xenon gas.
4) A mechanically activatable valve that opens a connection between a diffusion-resistant storage body **23** and the pump tubing **26**.
5) An electronically activatable valve that opens a connection between a diffusion-resistant storage body **23** and the pump tubing **26**.
6) Two one-sided (or a two-sided valve) between the pump tubing **26** and the storage body **23**. The one- or two-sided valve allowing the medium only to flow from the storage body 23 to the pump tubing **26** upon a pump action which draws the fluid medium into the pump tubing and pushes the medium back into the storage body upon a reverse pump action.

The connector body and/or the controller body may further include electrical components configured to measure various variables, such as temperature, sound, light, fluorescence, photoelectric effect, pressure, magnetism, flow, angular displacement, force, motion, inertia, electric impulses including ECG, EEG and EMG, glucose concentration, pO2, pCO2, SO2 or pH.

Fig. 6 illustrates three upstream locations **28**, **29**, **30** providing access for fluid so that various electrical components housed in the connector body **7** is capable of measuring different aspects related to the treatment.

In other embodiments, the electronic sensor converter is an analog-to-digital converter or a digital-to-analog converter and preferably a high-speed converter type, suitable for real-time data conversion and transmission. The electronic sensor converter can be controlled by means of a small computer such as a microcontroller or a microprocessor.

The internal design of the connector body **7** may prevent leakage of liquids inside the connector body **7**, and the connector body may include a liquid draining system, e.g. including a leakage connector **31**.

The connector body **7** may include its own power supply in the form of internal batteries, or it may be powered by the controller body.

Fig. 7 illustrates schematically the function of the junction **10** which forms an intersection between the first interfacing means **9** and the first fluid communication interface **11**. The junction **10** is located in the connector body **7**, and provides fluid communication from a selected one of the first interfacing means **9** and the first fluid communication interface **11** and the inflatable member such that the inflatable member can be expanded either manually via the first interfacing means or automatically via the first fluid communication interface.

### EXAMPLE 1

Operation of a device of the disclosure

The device is inserted into the descending aorta. The device has a display and speaker.

During operation, it turns out that the device has a fault in the automatically operated inflation means which is a power controlled pump. That means that the second interfacing means are unavailable for inflation or deflation of the inflation member.

The device informs the user of the status of the device through a display and by use of a speaker. In that way, the user is instructed to inflate the inflation member via the first interfacing means. The visual and oral instructions constitute in this case at part of the instruction set for manually operated inflation. In response, the user takes a syringe and connects it to the system and manually increases the filling of the inflation member while observing an inflation parameter via the human interface of the device. The device informs the user with a GREEN symbol on the display and a confirmatory sound via the speaker once the inflation has been reached. Again, the green symbol and confirmatory sound constitute a part of the instruction set for manually operated inflation.

Subsequently, the user decreases the filling of the balloon by way of a pre-filled syringe, and the first interfacing means while observing an inflation parameter via the human interface of the device. The user is informed with a GREEN symbol on the display and a confirmatory sound via the speaker once the deflation has been reached. Again, the green symbol and confirmatory sound constitute a part of the instruction set for manually operated inflation.

### EXAMPLE 2

Operation of a device of the disclosure

The device is inserted into the descending aorta. The device has a display and speaker. The user operates the second interfacing means connected to a second power-controlled inflation means. This operation is carried out via an instruction set for automatically operated inflation which in this case is constituted by control codes for the power controlled pump.

The user has selected a partial inflation of the inflation member to reach a targeted inflation parameter, here in the form of a set subject blood pressure.

The device informs the user of the status of the balloon and the blood pressure via a display.

The power controlled inflation means fail due to a part fault.

The device informs the operator through the human interface and instructs the user to increase the filling of the balloon via the first interfacing means. The user instructions constitute a part of the instruction set for manually operated inflation. The user takes a pre-filled syringe and connects it to the system and manually increases the filling of the balloon while observing the blood pressure until a desired pressure has been reached. The device informs the user with a GREEN symbol and a confirmatory sound via the speaker once the desired inflation status has been achieved. The green symbol and confirmatory sound constitute a part of the instruction set for manually operated inflation.

Subsequently, the device informs the user that the filling should be decreased. This information is provided to the user via a RED symbol on the display and via an alarm sound through a speaker. The red symbol and confirmatory sound constitute a part of the instruction set for manually operated inflation. In response, the user decreases the filling of the balloon by way of the pre-filled syringe, and the first interfacing means while observing the blood pressure. When the intended blood pressure has been reached, the user is informed with a BLUE okay symbol on the display and a confirmatory sound from a speaker. The blue symbol and confirmatory sound constitute a part of the instruction set for manually operated inflation.

Numbered list of a first alternative aspect:
1. An endovascular device (1) for providing at least partial occlusion in a blood vessel in a subject, the device comprising:
   - an elongated body (2) extending between a proximal end (3) and a distal end (4), the distal end being insertable into the blood vessel,
   - an inflatable member (6) formed about the elongated body (2) and configured to expand upon receipt of a fluid medium from an inflation means,
   - a sensor for sensing an occlusion parameter in the blood vessel*,*
   - an inflation conduit (16) providing fluid communication between the inflatable member and an inflation means,
   - a connector body (7), and
   - a controller body (8),
   wherein:
   - the connector body is attachable to the controller body,
   - the connector body is in fixed connection with the elongated body, and
   - the controller body comprises the inflation means.
2. The device according to embodiment 1, wherein at least a part of the connector body is sterilized by a first sterilization process and at least a part of the controller body is sterilized by a second sterilization process different from the first sterilization process.
3. The device according to any of embodiments 1 or 2, wherein the connector body (7) forms a first electric communication interface (12) configured to electrically communicate with a second electric communication interface (13) provided in the controller body (8).
4. The device according to any of embodiments 1-3, wherein the connector body forms a first fluid communication interface (11) configured to communicate fluid with a second fluid communication interface (25) provided in the controller body.
5. The device according to embodiments 3 and 4, wherein the first electric communication interface and the first fluid communication interface are arranged to form a first mutual connection interface in the connector body, wherein the second electric communication interface and the second fluid communication interface are arranged to form a second mutual connection interface in the controller body, and wherein the first and second mutual connection interfaces are configured for establishing both electrical communication and fluid communication by joining the connector body and the controller body.
6. The device according to embodiment 5, wherein the electrical and fluid communications established by joining the connector body and the controller body are established simultaneously.

Numbered list of a second alternative aspect
1. An endovascular device (1) for providing at least partial occlusion in a blood vessel in a subject, the device comprising:
   - an elongated body (2) extending between a proximal end (3) and a distal end (4), the distal end being insertable into the blood vessel,
   - an inflatable member (6) formed about the elongated body (2) and configured to expand upon receipt of a fluid medium from an inflation means,
   - a sensor for sensing an occlusion parameter in the blood vessel*,*
   - an inflation conduit (16) providing fluid communication between the inflatable member and a junction (10),
   - a first interfacing means (9) configured to connect to a first manually operated inflation means and being in fluid communication with the junction (10);
   - a second interfacing means (11) configured to connect to a second power controlled inflation means and being in fluid communication with the junction (10), and
   - an electronic control unit (22) configured to receive the occlusion parameter from the sensor and to provide an instruction set for manually operated inflation or for automatically operated inflation based on the occlusion parameter.
2. The device according to embodiment 1, comprising further comprising a connector body (7) and a controller body (8), wherein:
   - the connector body is attachable to the controller body,
   - the connector body is in fixed connection with the elongated body, and
   - the power controlled inflation means is formed in the controller body.
3. The device according to embodiment 2, wherein at least one of the first interfacing means (9) and the second interfacing means (11) are formed in the connector body (7).
4. The device according to embodiment 2 or 3, wherein at least one of the first interfacing means (9) and the second interfacing (11) means are formed in the controller body (8).
5. The device according to any of the preceding embodiments, comprising a storage body (23) for storage of the fluid medium.
6. The device according to any of embodiments 2-4 and 5, wherein the storage body is contained in the controller body (8).
7. The device according to any of the preceding embodiments, comprising an electronic sensor converter (21) configured to receive a fluid signal representing the occlusion parameter, to convert the fluid signal to an electrical signal, and to communicate the electrical signal to the electronic control unit (22).
8. The device according to any of embodiments 2-6 and 7, wherein the electronic sensor converter is contained in the connector body.
9. The device according to any of embodiments 7-8, wherein the fluid signal is received by the electronic sensor converter via a sensor conduit (15) extending in the elongated member between an upstream location (18) and the electronic sensor converter (21).
10. The device according to embodiment 9, wherein the upstream location is between the distal end (4) and the inflatable member (6).
11. The device according to any of embodiments 9-10, comprising a purge structure (14) allowing filling of the sensor conduit (15) with a propagation medium.
12. The device according to embodiment 11, wherein the purge structure comprises an external access port (14) configured to connect a propagation medium container.
13. The device according to embodiment 5 and 11-12, wherein the purge structure comprises a confluence configured to establish fluid communication between the sensor conduit and the storage body to allow purging with the fluid medium in the storage body (23).
14. The device according to embodiment 13, wherein the confluence is configured to be controlled by at least one or more of the following: A pressure difference between pressure in the inflation conduit and pressure in the sensor conduit, such that it allows a fluid flow between the inflation conduit and the sensor conduit upon a pressure difference above a first threshold value and such that it prevents fluid flow between the inflation conduit and the sensor conduit upon a pressure difference below the first threshold value; An electronic valve, including an electronic solenoid valve, pinch valve or tube pinch valve.
15. The device according to any of embodiments 2-14, wherein the connector body (7) forms a first electric communication interface (12) configured to electrically communicate with a second electric communication interface (13) provided in the controller body (8).
16. The device according to any of embodiments 2-15, wherein the connector body forms a first fluid communication interface (11) configured to communicate fluid with a second fluid communication interface (25) provided in the controller body.
17. The device according to embodiments 15 and 16, wherein the first electric communication interface and the first fluid communication interface are arranged to form a first mutual connection interface in the connector body, wherein the second electric communication interface and the second fluid communication interface are arranged to form a second mutual connection interface in the controller body, and wherein the first and second mutual connection interfaces are configured for establishing both electrical communication and fluid communication by joining the connector body and the controller body.
18. The device according to embodiment 17, wherein the electrical and fluid communications established by joining the connector body and the controller body are established simultaneously.
19. The device according to any of embodiments 15-18, wherein the controller body contains the electronic control unit (22) and is configured to receive the electrical signal from the connector body via the second electric communication interface.
20. The device according to embodiment 7 and any of embodiments 15-18, wherein the first electric communication interface (12) configured to electrically communicate the electrical signal from the electronic sensor converter (21).
21. The device according to any of the preceding embodiments, comprising a removable sheath narrowly enclosing the inflatable member while the inflatable member is in a non-inflated state.
22. The device according to any of the preceding embodiments, wherein the instructions for manual operation is a human comprehensible signal related to operation of the manually operated inflation means.
23. The device according to embodiment 21-22, wherein the electronic control unit is configured to detect whether the inflatable member is enclosed by the removable sheath.
24. The device according to any of embodiments 2-24, wherein at least a part of the connector body is sterilized by a first sterilization process and at least a part of the controller body is sterilized by a second sterilization process different from the first sterilization process.
25. A method for preparing a device according to any of embodiment 2-24 for use by connecting the connector body (7) to the controller body (8).
26. A method for effecting at least partial reduction of blood supply to a part of the body of a mammal, including a human being, the method comprising introduction of the endovascular device according to any one of embodiments 1-24 via its distal end (4) into a blood vessel of the mammal and advancing the inflatable member (6) to a position in the mammal's vascular system via which blood is supplied to the part of the body and inflating the inflatable member to a degree which reduces blood flow from the position in the vascular system to the part of the body.
27. The method according to embodiment 26, comprising selecting between use of a manual inflation process and an automatic inflation process, and based on the selection, using a manually operated inflation means based on the human signal or operating an automatically operated inflation means based on an electronic control signal.
28. The method according to any of embodiments 26-27, wherein the blood flow from the position in the vascular system is reduced to zero.
29. The method according to any of embodiments 26-29, wherein the position in the vascular system is in the descending aorta.
30. The method according to embodiment 29, wherein the at least partial reduction in blood flow results in redistribution of cardiac output to increase blood supply to the brain and/or heart.
31. The method according to embodiment 30, wherein the redistribution of the blood flow is at least one measure undertaken in order to provide resuscitation or suspended state in the patient.
32. The method according to embodiment 31, where also chest compressions are applied to the patient.
33. The method according to any one of embodiments 26-32, wherein the position in the vascular system is in an artery supplying one or more extremities or organs.
34. The method according to any one of embodiments 26-33, which is carried out to reduce or stop blood loss caused by arterial bleeding or arterial rupture.
35. The method according to any one of embodiments 26-34, wherein the distal end of the endovascular device is introduced via the femoral artery, preferably via a needle or cannula.
36. An endovascular device (1) for providing at least partial occlusion in a blood vessel in a subject, the device comprising:
   - an elongated body (2) extending between a proximal end (3) and a distal end (4), the distal end being insertable into the blood vessel,
   - an inflatable member (6) formed about the elongated body (2) and configured to expand upon receipt of a fluid medium from an inflation means,
   - a sensor for sensing an occlusion parameter in the blood vessel*,*
   - an inflation means,
   - a storage body (23) for storage of the fluid medium,
   - an inflation conduit (16) providing fluid communication between the inflatable member and the inflation means, and
   - a hose providing fluid communication between the inflation means and the storage body
      wherein:
      - at least one of the hose, the inflation conduit and the storage body is made from or comprises polypropylene, polyethylene, Polyethylene terephthalate, Polyvinyl chloride, Polyethylene laminated polyethylene terephthalate, or is made from a synthetic polymer covered with a layer of metal.

## Claims

1. An endovascular device (1) for providing at least partial occlusion in a blood vessel of a subject, the device comprising:
- an elongated body (2) extending between a proximal end (3) and a distal end (4), the distal end being insertable into the blood vessel;
- an inflatable member (6) formed about the elongated body (2) and configured to expand upon receipt of a fluid medium;
- a sensor configured to sense an occlusion parameter indicative of a degree of occlusion in the blood vessel;
- an inflation conduit (16) in fluid communication with the inflatable member (6);
- a connector body (7) in fixed connection with the elongated body (2);
- a controller body (8) comprising a power-controlled inflation means configured to supply the fluid medium to the inflatable member (6);
- a first interfacing means (9) comprised in the connector body (7) and forming an external connection port configured to connect to a first manually operated inflation means; and
- an electronic control unit (22) configured to receive an electrical signal representing an occlusion parameter and to provide an instruction set for automatically operated inflation based on the occlusion parameter,
where
- the inflatable member is a balloon made of an elastically deformable polymer material;
- the connector body (7) is attachable to the controller body (8);
- the connector body (7) comprises a first fluid communication interface (11), in fluid communication with the inflation conduit (16), and a first electrical communication interface (12), the first fluid communication interface (11) and the first electrical communication interface (12) being arranged to form a first mutual connection interface;
- the controller body (8) comprises a second fluid communication interface (25), in fluid communication with the power-controlled inflation means, and a second electrical communication interface (13), the second fluid communication interface (25) and the second electrical communication interface (13) being arranged to form a second mutual connection interface; and
- the first and second mutual connection interfaces are configured to establish, by joining the connector body (7) and the controller body (8), both electrical communication between the first and second electrical communication interfaces (12, 13) and fluid communication between the power-controlled inflation means and the inflatable member (6) through the first and second fluid communication interfaces (11, 25) and the inflation conduit (16),
- the device comprises an electronic sensor converter configured to receive a fluid signal from the elongated body, to convert the fluid signal to an electrical signal, and to communicate the electrical signal to the electronic control unit,
- the fluid signal indicates a pressure of fluid or a flow of fluid, and the electronic signal is an electrically expressed indication of that pressure or flow,
- the sensor converter communicates the electrical signal to the electronic control unit, and
- the electronic control unit is configured to provide a human signal which identifies when the degree of inflation is correct, the human signal being in the form of a visually recognizable signal, an audio signal, or a tactile signal.

2. The device according to claim 1, comprising a storage body (23) for storage of the fluid medium.

3. The device according to claim 2, wherein the storage body is contained in the controller body (8).

4. The device according to any of the preceding claims, wherein the fluid signal is received by the electronic sensor converter via a sensor conduit (15) extending in the elongated member between an upstream location (18) and the electronic sensor converter (21).

5. The device according to claim 4, wherein the upstream location is between the distal end (4) and the inflatable member (6).

6. The device according to any of claims 4-5, comprising a purge structure (14) allowing filling of the sensor conduit (15) with a propagation medium.

7. The device according to claim 6, wherein the purge structure comprises an external access port (14) configured to connect a propagation medium container.

8. The device according to claims 2 and one of claims 4-7, wherein the purge structure comprises a confluence configured to establish fluid communication between the sensor conduit and the storage body to allow purging with the fluid medium in the storage body (23).

9. The device according to claim 8, wherein the confluence is configured to be controlled by at least one or more of the following:
- a pressure difference between pressure in the inflation conduit and pressure in the sensor conduit, such that it allows a fluid flow between the inflation conduit and the sensor conduit upon a pressure difference above a first threshold value and such that it prevents fluid flow between the inflation conduit and the sensor conduit upon a pressure difference below the first threshold value;
- an electronic valve, including an electronic solenoid valve, pinch valve or tube pinch valve.

10. The device according to any of the preceding claims, wherein the electronic control unit (22) is configured to receive an electrical signal representing an occlusion parameter and to provide an instruction set for manually operated inflation based on the occlusion parameter.

11. The device according to any of the preceding claims, comprising a removable sheath narrowly enclosing the inflatable member while the inflatable member is in a non-inflated state.

12. The device according to claim 11, wherein the electronic control unit is configured to detect whether the inflatable member is enclosed by the removable sheath.
